# EUROPEAN PATENT SPECIFICATION

(11) **EP 2 065 029 B1**
(45) Date of publication and mention of the grant of the patent: **17.04.2013**
(21) Application number: 07121954.7
(22) Date of filing: 30.11.2007
(51) Int. Cl.: A61K 8/64, A61Q 19/08, C07K 5/10

(54) **Personal care and cosmetic composition containing tetrapeptides with the motifs GX1X2G, PX1X2P, or PX1X2K**
Körperpflege- und Kosmetikzusammensetzung mit Tetrapeptiden mit den Motiven GX1X2G, PX1X2P oder PX1X2K
Soins personnels et composition cosmétique contenant des tétrapeptides avec les motifs GX1X2G, PX1X2P, ou PX1X2K

(43) Date of publication of application: 03.06.2009
(73) Proprietor: Evonik Goldschmidt GmbH, 45127 Essen (DE)
(72) Inventor: Farwick, Mike, 45138, Essen (DE); Lersch, Peter, 46537, Dinslaken (DE)

(56) References cited:
- EP-A- 1 741 719
- WO-A-2007/143006
- WO-A-2007/146269
- DE-A1- 4 127 790
- DATABASE CA [Online] CHEMICAL ABSTRACTS SERVICE, COLUMBUS, OHIO, US; 2006, SHIBUYA, AKIRA ET AL: "Hair treatment preparations containing cyclic mercapto compounds and peptides" XP002485227 retrieved from STN Database accession no. 2006:1229450 & JP 2006 315978 A (SHOWA DENKO K. K., JAPAN) 24 November 2006 (2006-11-24)
- DATABASE CA [Online] CHEMICAL ABSTRACTS SERVICE, COLUMBUS, OHIO, US; 1982, "Cosmetics" XP002494421 retrieved from STN Database accession no. 1982:168539 & JP 57 002214 A (KANEBO COSMETICS, INC., JAPAN; MITSUBISHI CHEMICAL INDUSTRIES CO., LTD) 7 January 1982 (1982-01-07)
- DATABASE CA [Online] CHEMICAL ABSTRACTS SERVICE, COLUMBUS, OHIO, US; 1982, "Cosmetics containing peptides" XP002494422 retrieved from STN Database accession no. 1982:187106 & JP 57 002213 A (KANEBO COSMETICS, INC., JAPAN; MITSUBISHI CHEMICAL INDUSTRIES CO., LTD) 7 January 1982 (1982-01-07)

## Description

The application relates to personal care and cosmetic compositions containing special tetrapeptides with the motifs GX¹X²G, PX¹X²P, or PX¹X²K which are useful for treating visible signs of aging including wrinkles, stretch marks and dark circles.

### BACKGROUND OF THE INVENTION

The first visible signs of aging are usually found on the skin: fine lines and wrinkles, age spots, dryness, red blotches, sagging and flaccid skin can be among the first signs of skin aging. Frizzyness, dullness and loss of hair are also well-known symptoms. Numerous skin or hair care products are available to consumers for treatment or prevention of these signs and conditions that are caused by various external sources of stress, including, for example mechanical stress, atmospheric pollution, sun exposure (photoaging), and contact with household and other chemicals. This is, of course, in addition to further natural stresses such as diet, acne, and general aging.

Skin is perpetually exposed to these stresses, which result in visible signs of skin aging and damage, such as dryness and wrinkling, thinning of the skin and other histological changes. As the skin ages, there is a reduction in protein synthesis, an increase in proteolysis, a general loss of moisture and a general disruption of the skin barrier, connective tissue and cohesion.

Many compounds have been described as being useful for improving skin appearance and physiology, including reducing fine lines, wrinkles, dryness and other symptoms associated with aged or photodamaged skin. Many compositions are available on the world wide market. But improvement is always desirable.

Retinoids, alpha and beta-hydroxy acids, flavonoids and isoflavones are proposed to act against acne and wrinkles, to speed cell renewal and to correct hormonal imbalances. These remedies are often fraught with problems, such as instability (retinoids, flavonoids), irritation (retinoids, hydroxyacids) and potential side effects (phytoflavones).

International patent application No. WO 00/43,417 discloses the use of certain peptides and derivatives as cosmetics or pharmaceutical compositions for the regulation of impaired immunologic functions and inflammation of the skin. The peptides and derivatives described have between 2 and 5 amino acids in length. These peptides and derivatives are said to act by reducing the tissue concentrations of IL-6 and IL-8 to levels close to those observed in young tissues. Exemplified peptides include N-Palmitoyl-Pro-Arg, N-Palmitoyl-Thr-Lys-Pro-Arg, Arg-Lys-Pro-Arg and N-Palmitoyl-Gly-Gln-Pro-Arg. While combinations of various peptides in accordance with WO 00/43,417 are possible, the reference does not specifically identify a special common motif of a tripeptide and a tetrapeptide. See also U.S. Pat. Nos. 6,620,419 and 6,492,326 disclosing cosmetic and personal care product incorporating, inter alia, certain tetrapeptides.

There is also a cosmetic product under the trade name EYELISS currently on the market to treat bags under the eyes. This product of Sederma includes a mixture of N-Palmitoyl-Gly-Gln-Pro-Arg and the dipeptide Val-Trp. This dipeptide has no significant collagen stimulating activity and the combination does not exhibit any enhancement in this property over the levels provoked by the use of the tetrapeptide alone. See also JP 07/324,097 and JP 08/311,098 to Daicel Chem, Ind. Ltd., as well as Kawamoto Takafumi et al., Chemical Abstracts, Vol. 124, No. 19, 6 May 1996 (Abstract No. 261765).

More examples for the use of amino acids as moisture keeping compounds in cosmetic compositions are described in the US patents 4,772,591, US 3,849,576, US 5,135,913, US 4,859,653 and US 4,760,051 as well as in the European patent EP-B 0 070 048. Specific vitamins and amino acids are described either alone or in a wide variety of combinations. They are reported to penetrate the skin in order to keep the moisture and promote the homeostasis of the skin and the cell renewal.

In US-Patent 5,254,331 another skin care product is described containing a protein-amino-acid-vitamin-nucleotide-complex. This complex consists of propylene glycol, serum proteins, niacin amid, water, adenosine phosphate and Arg. The complex should promote the regeneration of epidermal cells and permanently enhance the moisture content of the skin.

Another example of the recent use of amino acids in skin care products is described in US patent 6,974,799 B2 where a combination of certain tetrapeptides with GHK-tripeptides may have beneficial effects on the visible signs of aging in human skin.

However, in spite of recent research in the field of amino acid compounds for the treatment of skin and hair aging no special tetrapeptides have been named that really show skin improvement in contrast to other tetrapeptides.
Thus, it is the object of the present invention to identify active ingredients for personal care and skin care products among the huge variety of tetrapeptides which can provide relief from one or more of the cited symptoms of hair and skin aging. Apart from that, the personal care and skin care composition should be easy to produce, formulate and handle in view of the related known products of the cited prior art.

Surprisingly, it has now been found that cosmetic compositions comprising special tetrapeptides with the special motifs GX¹X²G, PX¹X²P, or PX¹X²K show a bioactivity and can enhance the recovery of lines, reduce stretch marks, inflammation and micro damages of the skin thus helping to reduce the visible signs of aging. Furthermore, the inventive compositions can show surprising benefits to the treated skin in adding resiliency back to the skin.

Therefore, subject of the present invention are topical personal care and skin care compositions comprising:
a) a safe and effective amount of a tetrapeptide selected from the group of tetrapeptides with the motif GX¹X²G, PX¹X²P, or PX¹X²K, derivatives thereof, and mixtures thereof;
b) a safe and effective amount of at least one additional active ingredient selected from the group consisting of hydroxy acids, radical scavengers, UV absorbers, vitamins, barrier lipids, desquamatory actives, retinoids, anti-acne actives, tanning actives, anti-cellulite actives, anti-inflammatory actives, antimicrobial actives, antifungal actives, skin lightening agents, skin energizing agents, skin healing agents, moisturizing actives, and mixtures thereof; and
c) a dermatologically acceptable carrier.

In one aspect, the present invention relates to topical compositions containing tetrapeptides having the above stated motifs GX¹X²G, PX¹X²P, or PX¹X²K, formulated in combination with at least one additional skin care active or additional ingredient which includes a dermatologically acceptable carrier.

Certain aspects of the present invention also relate to methods of using such compositions to improve the state and appearance of human skin and to prevent and/or reduce the visible signs of aging. These methods generally consist in topically applying the composition to the skin when needed, in the amount and at the frequency best suited for the purpose. Methods of preventing, delaying the onset, or treating a skin condition are also contemplated.

All publications cited herein are hereby incorporated by reference in their entirety.

The cosmetic compositions of the present invention contain specific tetrapeptides, particularly tetrapeptides with one of the motifs GX¹X²G, PX¹X²P, or PX¹X²K and derivatives and mixtures thereof. These are collectively referred to herein as "tetrapeptides" unless the context or the explicit statements indicate otherwise. One or more additional ingredient(s), including one or more dermatologically acceptable carrier(s) are also preferably used in these compositions.

A "tetrapeptide" in accordance with the present invention is a compound that includes an uninterrupted sequence of four amino acids within its structure. These are indicated herein using a traditional one letter convention or three letter convention shown in the following in brackets from left (N-terminal end) to right (C-terminal end). In this nomenclature for example, G (Gly) is glycine, K (Lys) is lysine, and P (Pro) is proline.

The term "amino acid" as employed herein includes and encompasses all of the naturally occurring amino acids, both in the D- or L-configuration if optically active, and the known non-native, synthetic, and modified amino acids, such as homocysteine, ornithine, norleucine and p-valine. A list of non natural amino acids may be found in The Peptides, Vol. 5 (1983), Academic Press, Chapter VI, by D. C. Roberts and F. Vellaccio. The amino acids in the peptides of the present invention may be present in their natural L-configuration, unnatural D-configuration, or as a racemic mixture.

Derivatives are also considered to be encompassed by the term "tetrapeptides" in accordance with the present invention. Derivatives include derivatives of the substituted and rearranged tetrapeptides with one of the amino acid motifs GX¹X²G, PX¹X²P, or PX¹X²K described herein. These derivatives include, inter alia, acyl-derivatives, which are tetrapeptides substituted with one or more straight-acyl chain or branched-acyl chain, long or short acyl chain, saturated or unsaturated, substituted with a hydroxy, amino, amino acyl, sulfate or sulfide group or unsubstituted having from 1 to 29 carbon atoms. N-acyl-derivatives include those acyl groups which can be derived from acetic acid, capric acid, lauric acid, myristic acid, octanoic acid, palmitic acid, stearic acid, behenic acid, linoleic acid, linolenic acid, lipoic acid, oleic acid, isostearic acid, elaidoic acid, 2-ethylhexaneic acid, coconut oil fatty acid, tallow fatty acid, hardened tallow fatty acid, palm kernel oil fatty acid, lanolin fatty acid and the like. Preferable examples of the acyl group include an acetyl group, a palmitoyl group, an elaidoyl group, a myristyl group, a biotinyl group and an octanoyl group. These may be substituted or unsubstituted. When substituted, they are preferably substituted with hydroxyl or, sulphur containing groups such as, without limitation SO₃H, SH or S--S.

The term "dermatologically acceptable", as used herein, means that the compositions or components described are suitable for use in contact with human skin without risk of toxicity, incompatibility, instability, allergic response, and the like.

"Signs of skin aging" include, but are not limited to, all outward visibly and tactilely perceptible manifestations as well as any other macro or micro effects due to skin aging. Such signs may be induced or caused by intrinsic factors or extrinsic factors, e.g., chronological aging and/or environmental damage. These signs may result from processes which include, but are not limited to, the development of textural discontinuities such as wrinkles and coarse deep wrinkles, skin lines, crevices, bumps, large pores (e.g., associated with adnexal structures such as sweat gland ducts, sebaceous glands, or hair follicles), or unevenness or roughness, loss of skin elasticity (loss and/or inactivation of functional skin elastin), sagging (including puffiness in the eye area and jowls), loss of skin firmness, loss of skin tightness, loss of skin recoil from deformation, discoloration (including undereye circles), blotching, shallowness, hyperpigmented skin regions such as age spots and freckles, keratoses, abnormal differentiation, hyperkeratinization, elastosis, collagen breakdown, and other histological changes in the stratum corneum, dermis, epidermis, the skin vascular system (e.g., telangiectasia or spider vessels), and underlying tissues, especially those proximate to the skin.

All terms such as "skin aging," "signs of skin aging," "topical application," and the like are used in the sense in which they are generally and widely used in the art of developing, testing and marketing cosmetic and personal care products.

The term "cosmetic composition" or more briefly just "composition" in accordance with the present invention relates to a formulation that can be used for cosmetic purposes, purposes of hygiene or as a basis for delivery of one or more pharmaceutical ingredients. It is also possible that these formulations are used for two or more of these same purposes at one time. A medicated dandruff shampoo, for example, has pharmacological properties and is used as a personal care product to provide clean hair. At a minimum, these compositions include a tetrapeptide, analogs or derivatives thereof. These compositions may also include additional ingredients such as a dermatologically acceptable carrier.

"Cosmetics," as used herein, include without limitation, lipstick, mascara, rouge, foundation, blush, eyeliner, lipliner, lip gloss, facial or body powder, sunscreens and blocks, nail polish, mousse, sprays, styling gels, nail conditioner, whether in the form of creams, lotions, gels, ointments, emulsions, colloids, solutions, suspensions, compacts, solids, pencils, spray-on formulations, brush-on formulations and the like. Personal care products include, without limitation, bath and shower gels, shampoos, conditioners, cream rinses, hair dyes and coloring products, leave-on conditioners, sunscreens and sunblocks, lip balms, skin conditioners, hair sprays, soaps, body scrubs, exfoliants, astringents, depilatories and permanent waving solutions, antidandruff formulations, antisweat and antiperspirant compositions, shaving, preshaving and after shaving products, moisturizers, deoderants, cold creams, deodorants, cleansers, skin gels, rinses, whether in solid, powder, liquid, cream, gel, ointment, lotion, emulsions, colloids, solutions, suspensions, or other form. Pharmaceutical preparations in accordance with the present invention include, without limitation, carriers for dermatological purposes, including topical and transdermal application of pharmaceutically active ingredients. These can be in the form of gels, patches, creams, nose sprays, ointments, lotions, emulsions, colloids, solutions, suspensions, powders and the like.

The tetrapetide of the composition of the present invention is according to a preferred embodiment a tetrapeptide GX¹X²G, PX¹X²P, or PX¹X²K where X¹ and X² are different amino acids or both are P.

Furthermore, the tetrapeptide of the present invention preferably is selected from the group of tetrapeptides with the motifs GX¹X²G, PX¹X²P, or PX¹X²K where X¹ and X² are different amino acids or both are P, selected from the group consisting of E, G, P, and K.

The tetrapeptides of the present invention are preferably used in amounts from about 1 ppm (0.0001% w/w also referred to herein as "weight percent", "weight %" or simply by weight) to about 1000 ppm (0.1% w/w), preferably from about 2 ppm to about 250 ppm (0.025% w/w), and most preferably from about 10 ppm to about 100 ppm by weight of the composition.

Preferred embodiments include topical compositions wherein the tetrapeptide is selected from the group of GEPG (SEQ ID NO: 1), GPPG (SEQ ID NO: 2), GEKG (SEQ ID NO: 3), PGPP (SEQ ID NO: 4), and/or PKEK (SEQ ID NO: 5).

More preferred embodiments include N-acyl-GEPG (SEQ ID NO: 6) peptide, N-acyl-GPPG (SEQ ID NO: 7) peptide, N-acyl-GEKG (SEQ ID NO: 8) peptide, N-acyl-PGPP (SEQ ID NO: 9) peptide and/or N-acyl-PKEK (SEQ ID NO: 10) peptide, most preferably N-acyl is Npalmitoyl, N-lauryl or N-myristyl.

It has been unexpectedly found that the combination of selected tetrapeptides, as well as their analogs and derivatives, can provide significant advantages compared to products produced from other small peptides, either alone or in combination. It is believed that the special motifs of tetrapeptides according to the present invention have the ability in common to enhance the sythesis of collagen, fibronectin, elastin, and/or hyaluronic acid or reduce the degradation of these molecules. Thus they are acitve in enhancing the appearance and/or structure of the treated skin. In contrast to the employment of a randomly chosen tetrapeptide as described in the prior art it is believed that the special tetrapeptides of the present inveniton due to their motifs GX¹X²G, PX¹X²P, or PX¹X²K show better results in activation of skin synthesis of vital compounds and can stimulate cellular immuno system defensives. Furthermore, for the tetrapeptide PKEK (SEQ ID NO: 5) experiments have shown an inhibition of UV-induced expression of proinflammatory cytokines. Thus, the tetrapeptide can help to calm UV-irradiated skin.

In another preferred embodiment of the present invention the topical composition further comprises a GAGP (SEQ ID NO: 11) tetrapeptide.
It has been surprisingly found that the mixture of one or more of the tetrapeptides with the special motif GX¹X²G, PX¹X²P, or PX¹X²K with the tetrapeptide GAGP show even better results as anti-aging active ingredient in cosmetic compositions compared to the corresponding mixture of one or more of the tetrapeptides with the special motif GX¹X²G, PX¹X²P, or PX¹X²K without addition of the tetrapeptide GAGP.

In addition to the special tetrapeptides, analogs and/or derivatives thereof, the compositions of the invention comprises at least one of various other and additional ingredients, which may be active, functional, conventionally used in cosmetic, personal care or otherwise. Of course, the choice of specific additional ingredients depends on the specific application and product formulation. Also, the distinction between an "active" ingredient and an "inactive ingredient" is artificial and dependent on the specific application and product type. A substance that is an "active" ingredient in one application or product may be a "functional" ingredient in another, and vice versa. A particular ingredient might provide substantivity in one formulation, facilitate transdermal application in another, and merely provide proper viscosity in a third. Which of these is functional and which is active is subject to debate. But, regardless of the outcome, the material in question would qualify as an additional ingredient in accordance with the present invention.

Thus, the composition of the invention comprises at least one additional ingredient, which provides some benefit to the object of the composition. Such additional ingredients may include one or more substances such as, without limitations, cleaning agents, hair conditioning agents, skin conditioning agents, hair styling agents, antidandruff agents, hair growth promoters, perfumes, sunscreen and/or sunblock compounds, pigments, moisturizers, film formers, hair colors, make-up agents, detergents, pharmaceuticals, thickening agents, emulsifiers, humectants, emollients, antiseptic agents, deodorant actives, dermatologically acceptable carriers and surfactants.

In a preferred embodiment, where the composition is to be in contact with human keratinous tissue, the additional ingredients should be suitable for application to keratinous tissue, that is, when incorporated into the composition they are suitable for use in contact with human keratinous tissue (hair, nails, skin, lips) without undue toxicity, incompatibility, instability, allergic response, and the like within the scope of sound medical judgment. The CTFA Cosmetic Ingredient Handbook, Ninth Edition (2002) describes a wide variety of nonlimiting cosmetic and pharmaceutical ingredients commonly used in the skin care industry, which are suitable for use as additional ingredients in the compositions of the present invention. Non-limiting examples of these additional ingredient classes include: abrasives, absorbents, aesthetic components such as fragrances, pigments, colorings/colorants, essential oils, skin sensates, astringents, anti-acne agents, anti-caking agents, antifoaming agents, antimicrobial agents , antioxidants, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition, opacifying agents, pH adjusters, propellants, reducing agents, sequestrants, skin bleaching and lightening agents (e.g., hydroquinone, kojic acid, ascorbic acid, magnesium ascorbyl phosphate, ascorbyl glucosamine), skin-conditioning agents (e.g., humectants, including miscellaneous and occlusive), skin soothing and/or healing agents (e.g., panthenol and derivatives (e.g., ethyl panthenol), aloe vera, pantothenic acid and its derivatives, allantoin, bisabolol, and dipotassium glycyrrhizinate, skin treating agents, thickeners, and vitamins and derivatives thereof.

The compositions of the present invention generally contain at least one additional ingredient. The compositions of the present invention may contain a plurality of additional ingredients as well.

In any embodiment of the present invention, however, the additional ingredients useful herein can be categorized by the benefit they provide or by their postulated mode of action. However, it is to be understood that the additional ingredients useful herein can in some instances provide more than one benefit or operate via more than one mode of action. Therefore, classifications herein are made for the sake of convenience and are not intended to limit the additional ingredients to that particular application or applications listed.

### Desquamation Actives

A safe and effective amount of a desquamation active may be added to the compositions of the present invention, more preferably from about 0.1% to about 10%, even more preferably from about 0.2% to about 5%, also preferably from about 0.5% to about 4%, by weight of the composition. Desquamation actives enhance the skin appearance benefits of the present invention. For example, the desquamation actives tend to improve the texture of the skin (e.g., smoothness). One desquamation system that is suitable for use herein contains as active ingredients a combination at least one anionic polyamino acid matrix, at least one polysaccharide and optionally one or more osmoprotectants for topical application and is described in U.S. Pat. No. 20060275238 by Blasko-Begoihn et al.

### Enzymes, Enzyme Inhibitors and Enzyme Activators (Coenzymes)

The compositions of the present invention may contain a safe and effective amount of one or more enzymes, enzyme inhibitors or enzyme activators (coenzymes). Examples of enzymes are lipases, proteases, catalase, superoxide-dismutase, amylases, glucuronidases, lipoxygenases, peroxidases, in particular glutathione peroxidase or lactoperoxidase, ceramidases, hyaluronidases. All of these enzymes may be obtained by extraction or by fermentation biotechnology processes. Examples of enzyme inhibitors include trypsine inhibitors, Bowmann Birk inhibitor, chymotrypsin inhibitors, botanical extracts with or without tannins, flavonoids, stilbenes, hydroxystilbenes, quercetin which inhibit enzymatic activity. Enzyme activators and coenzymes include Coenzyme A, coenzyme Q10 (ubiquinone), glycyrrhizidine, berberine, chrysine.

### Botanical Extracts and Marine Extracts

The compositions of the present invention may contain a safe and effective amount of one or more extracts obtained from botanical or marine sources. These extracts may be obtained by standard extraction processes, and be used in powder, paste, balm, oil, or liquid (i.e. solution) form. These botanical and marine extracts possess various properties well known in cosmetic usage and may be advantageously combined with the special tetrapeptides object of this patent: soothing and anti-inflammatory, enzyme inhibition, moisturizing, anti-wrinkle, hormone replacement, anti-oxidant, emollient, seboregulating, anti-hairloss, hair growth promoting, anti-cellulite, skin healing, skin whitening, lipolytic, tanning, anti-microbial and the like.

### Anti-Acne Actives

The compositions of the present invention may contain a safe and effective amount of one or more anti-acne actives. Examples of useful anti-acne actives include sphingoidbases e.g. phytosphingosine or tetraacetylphytophingosine, retinoids e.g. isotretinoin, resorcinol, sulfur, salicylic acid, benzoyl peroxide, erythromycin, zinc, etc.

### Anti-Wrinkle Actives/Hydroxy Acids

The compositions of the present invention may further contain a safe and effective amount of one or more anti-wrinkle actives. Exemplary anti-wrinkle actives suitable for use in the compositions of the present invention include sulfur-containing D and L amino acids and their derivatives and salts, particularly the N-acetyl derivatives, a preferred example of which is N-acetyl-DL-Methionine; thiols, e.g. ethane thiol; hydroxy acids (e.g., alpha-hydroxy acids such as lactic acid and glycolic acid or beta-hydroxy acids such as salicylic acid and salicylic acid derivatives such as the dodecylamide; stilbenes; hydroxystilbenes; hyaluronic acid; flavonoids; xanthones; Beta-Glucans; Skleroglucan; triterpenoid acids (e.g. arjunolic acid, ursolic acid); turmeric oil; xymeninic acid; creatine; sphingolipids such as salicycloyl-phytosphingosine, phytosphingosine, sphingosine, sphinganine and their derivatives; phytic acid; lipoic acid; lysophosphatidic acid; skin peel agents (e.g., phenol and the like); vitamin B.sub.3 compounds and retinoids which enhance the keratinous tissue appearance benefits of the present invention, especially in regulating keratinous tissue condition, e.g., skin condition.

### Vitamin Compounds and Antioxidants

The compositions of the present invention may contain a safe and effective amount of a vitamin or antioxidant compound. Tocopherol and its derivatives, ascorbic acid and derivatives, niacinamide and derivatives, alpha-lipoic acid, and panthenol are particularly preferred vitamin and anitoxidant comounds. Also, vitamin B₃ compounds are particularly useful for regulating skin condition. When vitamin B₃ compounds are present in the compositions of the instant invention, the compositions preferably contain from about 0.01% to about 50%, more preferably from about 0.1% to about 10%, even more preferably from about 0.5% to about 10%, and still more preferably from about 1% to about 5%, still more preferably from about 2% to about 5%, by weight of the composition, of the vitamin B₃ compound. Examples of suitable vitamin B.sub.3 compounds are well known in the art and are commercially available from a number of sources, e.g., the Sigma Chemical Company (St. Louis, Mo.); ICN Biomedicals, Inc. (Irvin, Calif.) and Aldrich Chemical Company (Milwaukee, Wis.).
Additionally, other vitamin compounds such as vitamine E are particularly preferred to be used in cosmetic compositions asscording to the invention.

The vitamin compounds may be included as the substantially pure material, or as an extract obtained by suitable physical and/or chemical isolation from natural (e.g. plant) sources.

### Retinoids

The compositions of the present invention may also contain a retinoid. As used herein, "retinoid" includes all natural and/or synthetic analogs of Vitamin A or retinol-like compounds which possess the biological activity of Vitamin A in the skin as well as the geometric isomers and stereoisomers of these compounds. The retinoid is preferably retinol, retinol esters (e.g., C.sub.2 -C.sub.22 alkyl esters of retinol, including retinyl palmitate, retinyl acetate, retinyl propionate), retinal, and/or retinoic acid (including all-trans retinoic acid and/or 13-cis-retinoic acid), more preferably retinoids other than retinoic acid. These compounds are well known in the art and are commercially available from a number of sources, e.g., Sigma Chemical Company (St. Louis, Mo.), and Boerhinger Mannheim (Indianapolis, Ind.). Other suitable retinoids are tocopheryl-retinoate [tocopherol ester of retinoic acid (trans- or cis-), adapalene {6-[3-(1-adamantyl)-4-methoxyphenyl]-2-naphthoic acid}, and tazarotene (ethyl 6-[2-(4,4-dimethylthio-chroman-6-yl)-ethynyl]nicotinate).
Preferred retinoids are retinol, retinyl palmitate, retinyl acetate, retinyl propionate, retinal and combinations thereof.

The retinoid may be included as the substantially pure material, or as an extract obtained by suitable physical and/or chemical isolation from natural (e.g. plant) sources. The retinoid is preferably substantially pure, more preferably essentially pure.

The compositions of this invention may contain a safe and effective amount of the retinoid, such that the resultant composition is safe and effective for regulating keratinous tissue condition, preferably for regulating visible and/or tactile discontinuities in skin, more preferably for regulating signs of skin aging, even more preferably for regulating visible and/or tactile discontinuities in skin texture associated with skin aging. The compositions preferably contain from or about 0.005% to or about 2%, more preferably 0.01% to or about 2%, retinoid. Retinol is preferably used in an amount of from or about 0.01% to or about 0.15%; retinol esters are preferably used in an amount of from or about 0.01% to or about 2% (e.g., about 1%); retinoic acids are preferably used in an amount of from or about 0.01% to or about 0.25%; tocopheryl-retinoate, adapalene, and tazarotene are preferably used in an amount of from or about 0.01% to or about 2%.

Where the compositions of the present invention contain both a retinoid and a Vitamin B.sub.3 compound, the retinoid is preferably used in the above amounts, and the vitamin B.sub.3 compound is preferably used in an amount of from or about 0.1% to or about 10%, more preferably from or about 2% to or about 5%.

The compositions of the present invention may include a safe and effective amount of an anti-oxidant/radical scavenger or an oxidizer/reducing agent. The anti-oxidant/radical scavenger or oxidizer/reducing agent is especially useful for providing protection against UV radiation which can cause increased scaling or texture changes in the stratum corneum and against other environmental agents which can cause skin damage. These compounds may also be useful in hair drying and other cosmetic applications.

A safe and effective amount of an anti-oxidant/radical scavenger or an oxidizer/reducing agent may be added to the compositions of the subject invention, preferably from about 0.1% to about 10%, more preferably from about 1% to about 5%, of the composition.

Anti-oxidants/radical scavengers such as ascorbic acid (vitamin C) and its salts, ascorbyl esters of fatty acids, ascorbic acid derivatives (e.g., magnesium ascorbyl phosphate, sodium ascorbyl phosphate, ascorbyl sorbate), tocopherol (vitamin E), tocopherol sorbate, tocopherol acetate, other esters of tocopherol, butylated hydroxy benzoic acids and their salts, peroxides including hydrogen peroxide, perborate, thioglycolates, persulfate salts, 6-hydroxy-2,5,7,8-tetramethylchroman-2-carboxylic acid (commercially available under the tradename TROLOX.RTM.), gallic acid and its alkyl esters, especially propyl gallate, uric acid and its salts and alkyl esters, sorbic acid and its salts, lipoic acid, amines (e.g., N,N-diethylhydroxylamine, amino-guanidine), sulfhydryl compounds (e.g., glutathione), dihydroxy fumaric acid and its salts, lycine pidolate, arginine pidolate, nordihydroguaiaretic acid, bioflavonoids, curcumin, lysine, 1-methionine, proline, superoxide dismutase, silymarin, tea extracts, grape skin/seed extracts, melanin, and rosemary extracts may be used. Preferred anti-oxidants/radical scavengers are selected from tocopherol sorbate and other esters of tocopherol, more preferably tocopherol acetate.

### Flavonoids

The compositions of the present invention may optionally contain a flavonoid compound. Flavonoids suitable for use in the present invention are flavanones selected from unsubstituted flavanones, mono-substituted flavanones, and mixtures thereof; chalcones selected from unsubstituted chalcones, mono-substituted chalcones, di-substituted chalcones, tri-substituted chalcones, and mixtures thereof; flavones selected from unsubstituted flavones, mono-substituted flavones, di-substituted flavones, and mixtures thereof; one or more isoflavones; coumarins selected from unsubstituted coumarins, mono-substituted coumarins, di-substituted coumarins, and mixtures thereof; chromones selected from unsubstituted chromones, mono-substituted chromones, di-substituted chromones, and mixtures thereof; one or more dicoumarols; one or more chromanones; one or more chromanols; isomers (e.g., cis/trans isomers) thereof; and mixtures thereof. By the term "substituted" as used herein means flavonoids wherein one or more hydrogen atom of the flavonoid has been independently replaced with hydroxyl, C1-C8 alkyl, C1-C4 alkoxyl, O-glycoside, and the like or a mixture of these substituents.

Examples of suitable flavonoids include, but are not limited to, unsubstituted flavanone, mono-hydroxy flavanones (e.g., 2'-hydroxy flavanone, 6-hydroxy flavanone, 7-hydroxy flavanone, etc.), mono-alkoxy flavanones (e.g., 5-methoxy flavanone, 6-methoxy flavanone, 7-methoxy flavanone, 4'-methoxy flavanone, etc.), unsubstituted chalcone (especially unsubstituted trans-chalcone), mono-hydroxy chalcones (e.g., 2'-hydroxy chalcone, 4'-hydroxy chalcone, etc.), di-hydroxy chalcones (e.g., 2',4-dihydroxy chalcone, 2',4'-dihydroxy chalcone, 2,2'-dihydroxy chalcone, 2',3-dihydroxy chalcone, 2',5'-dihydroxy chalcone, etc.), and tri-hydroxy chalcones (e.g., 2',3',4'-trihydroxy chalcone, 4,2',4'-trihydroxy chalcone, 2,2',4'-trihydroxy chalcone, etc.), unsubstituted flavone, 7,2'-dihydroxy flavone, 3',4'-dihydroxy naphthoflavone, 4'-hydroxy flavone, 5,6-benzoflavone, and 7,8-benzoflavone, unsubstituted isoflavone, daidzein (7,4'-dihydroxy isoflavone), 5,7-dihydroxy-4'-methoxy isoflavone, soy isoflavones (a mixture extracted from soy), unsubstituted coumarin, 4-hydroxy coumarin7-hydroxy coumarin, 6-hydroxy-4-methyl coumarin, unsubstituted chromone, 3-formyl chromone, 3-formyl-6-isopropyl chromone, unsubstituted dicoumarol, unsubstituted chromanone, unsubstituted chromanol, and mixtures thereof.

They can be synthetic materials or obtained as extracts from natural sources (e.g., plants). The naturally sourced material can also further be derivatized (e.g., an ester or ether derivative prepared following extraction from a natural source). Flavonoid compounds useful herein are commercially available from a number of sources, e.g., Indofine Chemical Company, Inc. (Somerville, N.J.), Steraloids, Inc. (Wilton, N.H.), and Aldrich Chemical Company, Inc. (Milwaukee, Wis.).

Mixtures of the above flavonoid compounds may also be used.

The herein described flavonoid compounds are preferably present in the instant invention at concentrations of from about 0.01% to about 20%, more preferably from about 0.1% to about 10%, and still more preferably from about 0.5% to about 5%.

### Anti-Inflammatory Agents

A safe and effective amount of an anti-inflammatory agent may be added to the compositions of the present invention, preferably from about 0.1% to about 10%, more preferably from about 0.5% to about 5%, of the composition. The anti-inflammatory agent enhances the skin appearance benefits of the present invention, e.g. such agents contribute to a more uniform and acceptable skin tone or color. The exact amount of anti-inflammatory agent to be used in the compositions will depend on the particular anti-inflammatory agent utilized since such agents vary widely in potency.

Anti-inflammatory agents can be classified into steroidal and non-steroidal agents. Specific steroidal and non-steroidal anti-inflammatory agents useful in the composition invention include, but are not limited to: bisabolol, allantoin, phytantriol, coenzyme Q10, liccorice extract, glycyrrhizidine and idebenone.

Mixtures of these anti-inflammatory agents may also be employed, as well as the dermatologically acceptable salts and esters of these agents.

### Anti-Cellulite Agents

The compositions of the present invention may also contain a safe and effective amount of an anti-cellulite agent. Suitable agents may include, but are not limited to, xanthine compounds like caffeine, theophylline, theobromine, and aminophylline.

### Tanning Actives

The compositions of the present invention may contain a tanning active. When present, it is preferable that the compositions contain from about 0.1% to about 20%, more preferably from about 2% to about 7%, and still more preferably from about 3% to about 6%, by weight of the composition, of dihydroxyacetone or erythrulose as an artificial tanning active.

Dihydroxyacetone, which is also known as DHA or 1,3-dihydroxy-2-propanone, is a white to off-white, crystalline powder.

The compound can exist as a mixture of monomers and dimers, with the dimers predominanting in the solid crystalline state. Upon heating or melting, the dimers break down to yield the monomers. This conversion of the dimeric form to the monomeric form also occurs in aqueous solution. Dihydroxyacetone is also known to be more stable at acidic pH values. See The Merck Index, Tenth Edition, entry 3167, p. 463 (1983), and "Dihydroxyacetone for Cosmetics," E. Merck Technical Bulletin, 03-304 110, 319 897, 180 588.

### Skin Lightening Agents

The compositions of the present invention may contain a skin lightening agent. When used, the compositions preferably contain from about 0.1% to about 10%, more preferably from about 0.2% to about 5%, also preferably from about 0.5% to about 2%, by weight of the composition, of a skin lightening agent. Suitable skin lightening agents include those known in the art, including kojic acid, arbutin, creatinine, turmeric oil, tetrahydrocurcurminoids, ascorbic acid and derivatives thereof, phytosphingosine, N-acetyl phytosphingosine, sphingosinephosphorylcholine and hydroquinone.

### Skin Soothing and Skin Healing Actives

The compositions of the present invention may comprise a skin soothing or skin healing active. Skin soothing or skin healing actives suitable for use herein include panthenoic acid derivatives including panthenol, dexpanthenol, ethyl panthenol, aloe vera, allantoin, bisabolol, and dipotassium glycyrrhizinate. A safe and effective amount of a skin soothing or skin healing active may be added to the present composition, preferably, from about 0.1% to about 30%, more preferably from about 0.5% to about 20%, still more preferably from about 0.5% to about 10%, by weight of the composition formed.

### Antimicrobial and Antifungal Actives

The compositions of the present invention may contain an antimicrobial or antifungal active. Such actives are capable of destroying microbes, preventing the development of microbes or preventing the pathogenic action of microbes. A safe and effective amount of an antimicrobial or antifungal active may be added to the present compositions, preferably, from about 0.001% to about 10%, more preferably from about 0.01% to about 5%, and still more preferably from about 0.05% to about 2%.

Examples of antimicrobial and antifungal actives include beta.-lactam drugs, guanidinium compounds, quinolone drugs, ciprofloxacin, norfloxacin, tetracycline, erythromycin, amikacin, 2,4,4'-trichloro-2'-hydroxy diphenyl ether, 3,4,4'-trichlorobanilide, phenoxyethanol, phenoxy propanol, phenoxyisopropanol, doxycycline, capreomycin, chlorhexidine, N-octyl lactamide, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole, tetracycline hydrochloride, erythromycin, zinc erythromycin, erythromycin estolate, erythromycin stearate, amikacin sulfate, doxycycline hydrochloride, capreomycin sulfate, chlorhexidine gluconate, chlorhexidine hydrochloride, chlortetracycline hydrochloride, oxytetracycline hydrochloride, clindamycin hydrochloride, ethambutol hydrochloride, metronidazole hydrochloride, pentamidine hydrochloride, gentamicin sulfate, kanamycin sulfate, lineomycin hydrochloride, methacycline hydrochloride, methenamine hippurate, methenamine mandelate, minocycline hydrochloride, neomycin sulfate, netilmicin sulfate, paromomycin sulfate, streptomycin sulfate, tobramycin sulfate, miconazole hydrochloride, ketaconazole, amanfadine hydrochloride, amanfadine sulfate, octopirox, parachlorometa xylenol, nystatin, tolnaftate, zinc pyrithione and clotrimazole.

Preferred examples of actives useful herein include those selected from salicylic acid, benzoyl peroxide, 3-hydroxy benzoic acid, glycolic acid, lactic acid, 4-hydroxy benzoic acid, acetyl salicylic acid, 2-hydroxybutanoic acid, 2-hydroxypentanoic acid, 2-hydroxyhexanoic acid, cis-retinoic acid, trans-retinoic acid, retinol, phytic acid, N-acetyl-L-cysteine, lipoic acid, azelaic acid, arachidonic acid, benzoylperoxide, tetracycline, ibuprofen, naproxen, hydrocortisone, acetominophen, resorcinol, phenoxyethanol, phenoxypropanol, phenoxyisopropanol, 2,4,4'-trichloro-2'-hydroxy diphenyl ether, 3,4,4'-trichlorocarbanilide, octopirox, lidocaine hydrochloride, clotrimazole, miconazole, ketoconazole, neocycin sulfate, and mixtures thereof.

### Sunscreen Actives and UV absobents

Exposure to ultraviolet light can result in excessive scaling and texture changes of the stratum corneum. Therefore, the compositions of the subject invention may optionally contain a sunscreen active. As used herein, "sunscreen active" includes both sunscreen agents and physical sunblocks. Suitable sunscreen actives may be organic or inorganic.

Inorganic sunscreens useful herein include the following metallic oxides; titanium dioxide, zinc oxide , zirconium oxide, iron oxide and mixtures thereof. When used herein, the inorganic sunscreens are present in the amount of from about 0.1% to about 20%, preferably from about 0.5% to about 10%, more preferably from about 1% to about 5%, by weight of the composition.

A wide variety of conventional organic sunscreen actives are suitable for use herein. Sagarin, et al., at Chapter VIII, pages 189 et seq., of Cosmetics Science and Technology (1972), discloses numerous suitable actives. A safe and effective amount of the organic sunscreen active is used, typically from about 1% to about 20%, more typically from about 2% to about 10% by weight of the composition. Exact amounts will vary depending upon the sunscreen or sunscreens chosen and the desired Sun Protection Factor (SPF).

### Moisturising Agents

The compositions of the present invention may contain a moisturising agent. A variety of these materials can be employed and each can be present at a level of from about 0.01% to about 20%, more preferably from about 0.1% to about 10%, and still more preferably from about 0.5% to about 7% by weight of the composition. These materials include, but are not limited to, amino acids and derivatives, creatine and derivatives, trimethylglycine, myoinositol, pyroglutamic acid and derivatives, taurine, urea, lactic acid, guanidine and derivatives, and hydroxy acids.

### Barrier Lipids

The compositions of the present invention can contain one or more barrier lipids. Preferred barrier lipids include but are not limited to ceramides, phytosphingosine, sphingosine, pseudoceramides, phospholipids, cholesterol, cholesteryl esters, free fatty acids and lysophospholipids.

### Structuring Agents

The compositions of the present invention, and especially the emulsions hereof, may contain a structuring agent. Structuring agents are particularly preferred in the oil-in-water emulsions of the present invention. Without being limited by theory, it is believed that the structuring agent assists in providing rheological characteristics to the composition which contribute to the stability of the composition. For example, the structuring agent tends to assist in the formation of the liquid crystalline gel network structures. The structuring agent may also function as an emulsifier or surfactant. Preferred compositions of this invention contain from about 0.1% to about 20%, more preferably from about 0.1% to about 10%, still more preferably from about 0.5% to about 9%, of one or more structuring agents.

Preferred structuring agents are those having an HLB of from about 1 to about 8 and having a melting point of at least about 45.degree. C.
The preferred structuring agents of the present invention are selected from stearic acid, palmitic acid, stearyl alcohol, cetyl alcohol, behenyl alcohol, stearic acid, palmitic acid, the polyethylene glycol ether of stearyl alcohol having an average of about 1 to about 5 ethylene oxide units, the polyethylene glycol ether of cetyl alcohol having an average of about 1 to about 5 ethylene oxide units, and mixtures thereof. More preferred structuring agents of the present invention are selected from stearyl alcohol, cetyl alcohol, behenyl alcohol, the polyethylene glycol ether of stearyl alcohol having an average of about 2 ethylene oxide units (steareth-2), the polyethylene glycol ether of cetyl alcohol having an average of about 2 ethylene oxide units, and mixtures thereof. Even more preferred structuring agents are selected from stearic acid, palmitic acid, stearyl alcohol, cetyl alcohol, behenyl alcohol, steareth-2, and mixtures thereof.

### Thickening Agent (Including Thickeners and Gelling Agents)

The compositions of the present invention can contain one or more thickening agents, preferably from about 0.1% to about 5%, more preferably from about 0.1% to about 4%, and still more preferably from about 0.25% to about 3%, by weight of the composition.

Nonlimiting classes of thickening agents include those selected from the following: carboxylic acid polymers,crosslinked polyacrylate polymers, polyacrylamide polymers, polysaccharides, gums, and other thickening and gelling agents which include materials which are primarily derived from natural sources like xanthan gum or cellulose and cellulose derivatives.

### Dermatologically-Acceptable Carrier

The compositions of the invention may be used in various cosmetic and/or personal care products, for example, skin care, hair care, nail care, facial and body care and sunscreen compositions, such as lotions, gels, sprays, and the like, hand cleaners, bath compositions, suntan oils, anti-perspirant compositions, perfumes and colognes, cold creams, hair sunscreen compositions, pre-shaves, deodorants, topical pharmaceutical ointments, skin moisturizers, facial cleansers, cleansing creams, skin gels, shampoos, hair conditioners, detergents, household cleaning products, make-up products, lipstick products, mascara, and hair coloring products. Therefore, in addition to any of the above cited skin care or hair care peptides and other actives, the cosmetic compositions described in the present invention may often include as an additional ingredient a dermatologically acceptable carrier. The form of the carrier and the final product resulting from the combination of the tetrapeptides with any additional active and with the carrier may be any of the following: liquids, gels, creams, water-in-oil and oil-in-water, and silicone emulsions, foams, and solids; they may be clear or opaque; and may be formulated as both aqueous and non-aqueous preparations, including but not limited to topical preparations.

To realize the invention in any of these physical forms, further substances, agents and compounds are useful although not always necessary such as conditioning agents, structuring agents and thickening agents. These compounds sometimes also have the role of adjuvant and sometimes the role of additional ingredient. Neither role excludes them from the present invention as being combined with the tetrapeptide or tetrapeptide mixtures of the invention and their derivatives.

The nature of the dermatologically acceptable carrier, the nature of the final product, and the methods of preparing those need not be described here in detail; many examples can be found in the available literatures.

In most instances, the additional ingredients will include a dermatologically acceptable carrier either alone or in combination with still other additional ingredients. The amounts of additional ingredients may range from about 99.5% to about 99.99999%, preferably from about 99.9% to about 99.9999%, more preferably from about 99.99% to about 99.999%, of the composition. In short, it is the balance of the composition. If carriers (either singularly, such as water, or complex cosolvents) are used, they may make up the entire balance of the compositions.

The carrier can be in a wide variety of forms. For example, emulsion carriers, including, but not limited to, oil-in-water, water-in-oil, water-in-oil-in-water, and oil-in-water-in-silicone emulsions, are useful herein.

Preferred carriers contain an emulsion such as oil-in-water emulsions, water-in-oil emulsions, and water-in-silicone emulsions. As will be understood by the skilled artisan, a given component will distribute primarily into either the water or oil/silicone phase, depending on the water solubility/dispersibility of the component in the composition. Oil-in-water emulsions are especially preferred.

Emulsions according to the present invention generally contain a solution as described above and a lipid or oil. Lipids and oils may be derived from animals, plants, or petroleum and may be natural or synthetic. Preferred emulsions also contain a humectant, such as glycerin. Emulsions will preferably further contain from about 0.01% to about 10%, more preferably from about 0.1% to about 5%, of an emulsifier, based on the weight of the carrier. Emulsifiers may be nonionic, anionic or cationic. Suitable emulsifiers are disclosed in, for example, McCutcheon's Detergents and Emulsifiers, North American Edition, pages 317-324 (1986).

### Emulsions

Preferred topical carriers include oil-in-water emulsions, having a continuous aqueous phase and a hydrophobic, water-insoluble phase ("oil phase") dispersed therein. An oil-in-water emulsion is described in detail hereinafter.

The preferred oil-in-water emulsion contains from about 25% to about 98%, preferably from about 65% to about 95%, more preferably from about 70% to about 90% water by weight of the topical carrier.

The oil phase can comprise any cosmetic oil or a mixture thereof. Examples of such oils include but are not limited to aliphatic hydrocarbons, such as liquid paraffin, squalene, vaseline and ceresin, vegetable oils, such as olive oil, almond oil, sesame oil, avocado oil, castor oil, cocoa butter and palm oil, animal oils, such as shark liver oil, cod liver oil, whale oil, beef tallow and butterfat, waxes such as beeswax, carnauba palm wax, spermaceti and lanolin, fatty acids, such as lauric acid, myristic acid, palmitic, acid, stearic acid, oleic acid, and behenic acid; aliphatic alcohols such as lauryl, stearyl, cetyl, and oleyl alcohol and aliphatic esters such as isopropyl, isocetyl, or octadecyl myristate, butyl stearate, hexyl laureate, diisopropyl ester of adipic acid, or diisopropyl sebacate. Preferred mono- or polyols for use in oil-alcohol lotions or oil-alcohol gel or alcohol gel include ethanol, isopropanol, propylene glycol, hexylene glycol, glycerol and sorbitol.

The topical compositions of the subject invention, including but not limited to lotions and creams, may contain a dermatologically acceptable emollient. Such compositions preferably contain from about 1% to about 50% of the emollient. As used herein, "emollient" refers to a material useful for the prevention or relief of dryness, as well as for the protection of the skin. A wide variety of suitable emollients are known and may be used herein. Sagarin, Cosmetics Science and Technology, 2nd Edition, Vol. 1, pp. 32-43 (1972), incorporated herein by reference, contains numerous examples of materials suitable as an emollient. A preferred emollient is glycerin. Glycerin is preferably used in an amount of from or about 0.001 to or about 30%, more preferably from or about 0.01 to or about 20%, still more preferably from or about 0.1 to or about 10%, e.g., 5%.

The compositions of the invention may also include a wide range of miscellaneous ingredients. Some suitable miscellaneous ingredients commonly used in the cosmetic and personal care industry are described in The CTFA Cosmetic Ingredient Handbook, (9.sup.th Ed., 2002), which is incorporated by reference herein. These ingredients will be used in amounts which are conventional.

In a preferred embodiment of the present invention the topical composition comprises from about 10 ppm to about 100 ppm, by weight of the composition, of either GEPG, GPPG, GEKG, PKEK, or PGPP; a dermatologically acceptable carrier and at least one additional active ingredient selected from
a) from about 0.05% to about 1.5%, by weight of the composition, of creatine;
b) from about 0.01% to about 1.0%, by weight of the composition, of ceramides and/or sphingolipids;
c) from about 0.01% to about 0.5%, by weight of the composition, of a hyaluronic acid and/or beta glucane;
d) from about 0.05% to about 1.0%, by weight of the composition, of arjunolic acid and/or turmeric oil.

Generally, typical formulations for personal care products and skin care products are widely known in the art and can for example be derived from the broschures of the manufacturers of the ingredients. Another source for information about possible formulations is for instance the anual "Kosmetik-Jahrbuch" (Editor: B. Ziolkowsky, Verlag für Chemische Industrie).

A typical example for an oil/water cream formulation is as follows:

| | |
|---|---|
| Ceteareth-25 | 2.0 % w/w |
| Glyceryl Stearate | 4.0 % w/w |
| Stearyl alcohol | 2.0 % w/w |
| Ethyl hexyl Stearate | 8.5 % w/w |
| Caprylic/Capric Triglyceride | 8.5 % w/w |
| Conservation means | 0.1 % w/w |
| Water ad | 100.0 % w/w |
| Tetrapeptide GX¹X²G, PX¹X²P, or PX¹X²K | 0.0001% w/w to 0.1% w/w |

The physical form of the compositions according to the invention is not important: creams, lotions, ointments, gels, emulsions, dispersions, solutions, suspensions, cleansers, foundations, anhydrous preparations (sticks, in particular lipsticks, body and bath oils), shower and bath gels and washes, shampoos and scalp treatment lotions, skin "essences", serums, adhesive or absorbent materials, transdermal patches, and powders can all incorporate the tetrapeptide, their analogs and derivatives thereof as well as combinations of these compounds with other additional ingredients.

The present invention also contemplates various uses. First, the invention contemplates the use of special tetrapeptides with the motif GX¹X²G, PX¹X²P, or PX¹X²K, in cosmetics or personal care products and their use for cosmetic and personal care purposes.
In particular, products produced in accordance with the present invention can be used to help mitigate the visible signs of aging, which include all outwardly visible and tactily perceptible manifestations as well as other macro or micro effects due to skin aging. Such signs may be induced or caused by intrinsic or extrinsic factors. The use in reducing these signs may be general or may be specific to use in reducing the visible signs of development of textural discontinuities, skin lines, crevices, bumps, large pores or skin unevenness or roughness, loss of skin elasticity, sagging, loss of skin firmness, loss of skin tightness, loss of skin recoil from deformation, discoloration, blotching, shallowness, hyperpigmented skin regions, keratoses, abnormal differentiation, hyperkeratinisation, elastosis, collagen breakdown, stretch marks, dark circles and the like. The use of these formulations for these cosmetic purposes may produce some extended change in the skin, but often produces a more transitory reduction in the visible manifestations of various conditions.

The compositions of the present invention are useful for preventing and/or reducing the visible signs of aging, and for improving the state of human skin or hair and its appearance. This includes preventive and curative treatment of the skin. For example, such methods are intended to thicken the various skin layers and tissues, preventing the thinning of the skin, reducing, preventing and/or retarding the appearance of wrinkles, improving firmness and elasticity of the skin, softening and/or smoothing lips, hair and nails, preventing and/or relieving itch, diminishing wrinkles and fine lines, diminishing stretch marks and dark circles, and/or stimulate collagen I synthesis.

This method of improving skin appearance involves topically applying to the skin or hair an effective amount of a composition of the present invention. The amount of the composition which is needed, the frequency of application and the duration period of use will depend on the amount of tetrapeptide with the motif GX¹X²G, PX¹X²p, or PX¹X²K, or derivatives thereof contained in the composition and on the specific combination with other additional ingredients, which can include, for example, pharmaceutically active agents, vitamins, alpha-hydroxy acids and the like, and the strength of the cosmetic effect desired.

Most advantageously, the compositions of the invention are applied to the skin or hair, once or preferably twice a day, over an extended period of time, at least one week, preferably one month, even more preferably 3 months, even more preferably for at least about six months, and still more preferably for at least about one year.

To practice the method, a composition in the form of a skin lotion, cream, gel, foam, ointment, paste, emulsion, spray, conditioner, tonic, cosmetic make-up, lipstick, foundation, nail polish, after-shave or the like, is applied to the skin and intended to stay there (leave-on). The composition can be applied manually, with the aid of spatulas, wipes or similar cosmetic tools. It can also be applied by the use of an occlusive or semi-occlusive patch, an adhesive or non-adhesive tissue.

In another embodiment of the present invention the topical composition can be used to apply a method of up-regulating the expression of fibronectin 1 in dermal tissue; here most preferably tetrapeptides of SEQ ID NO:2 or SEQ ID NO:4 are used as component a in the composition.
In yet another embodiment of the present invention the topical composition can be used to apply a method of up-regulating the expression of hyaluronic acid synthetase in dermal tissue; here most preferably tetrapeptides of SEQ ID NO:1 or SEQ ID NO:3 are used as component a in the composition.
In still another embodiment of the present invention the topical composition can be used to apply a method of up-regulating the expression of collagen I alpha-1 chain in dermal tissue; here most preferably tetrapeptides of SEQ ID NO:11 or a mixture of the tetrapeptides SEQ ID NO:1,2,3,11 are used as component a in the composition.
In still another embodiment of the present invention the topical composition can be used to apply a method of up-regulating the expression of collagen I alpha-2 chain in dermal tissue; here preferably tetrapeptides of SEQ ID NO: 1 or SEQ ID NO:4 and even more preferred a mixture of the tetrapeptides SEQ ID NO:1,2,3,11 are used as component a in the composition.
In still another embodiment of the present invention the topical composition can be used to apply a method of up-regulating the expression of collagen in dermal tissue; here preferably tetrapeptides of SEQ ID NO: 3 or SEQ ID NO:4 and even more preferred a mixture of the tetrapeptides SEQ ID NO:1,2,3,11 are used as component a in the composition.

The invention will now be described by reference to the following examples and figures which are merely illustrative and are not to be construed as a limitation of scope of the present invention.

### Brief description of the figures:

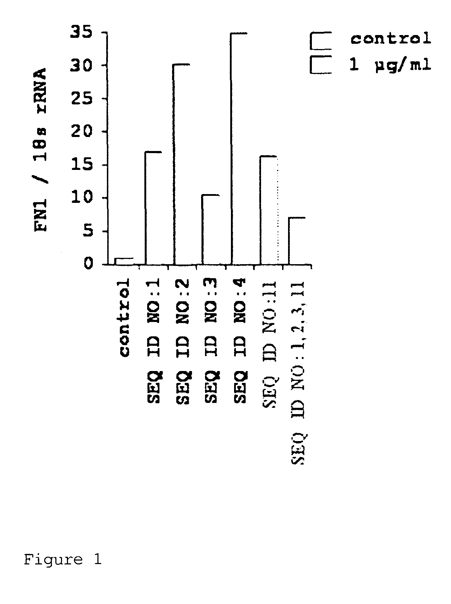

Figure 1: Fibronectin 1 (FN1) expression in human dermal fibroblasts (HDFs). HDFs were incubated with 1 µg/ml of the respective tetrapeptide (SEQ ID NO:1 - SEQ ID NO:4 and SEQ ID NO:11) or with a combination of all four tetrapeptides (SEQ ID NO:1,2,3,11). Controls received PBS. Cells were harvested after 24 hours incubation and RNA was isolated. Expression of FN1 was determined by quantitative PCR. 18s rRNA served as house keeping gene for normalization. Given are means of three independent experiments.

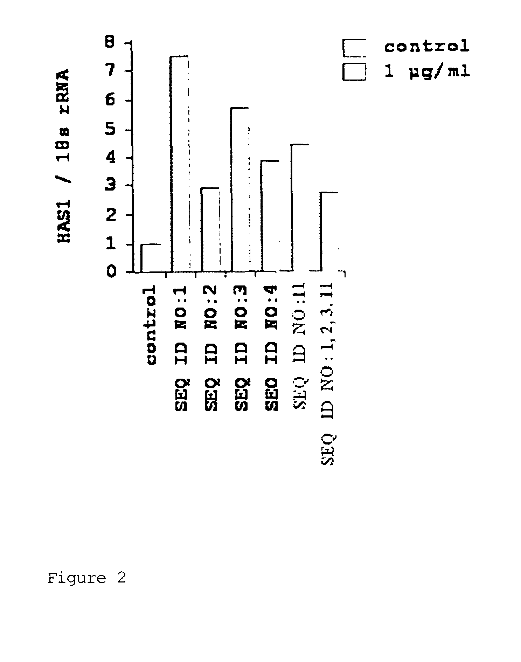

Figure 2: Hyaluronic acid synthetase 1 (HAS1) expression in human dermal fibroblasts (HDFs). HDFs were incubated with 1 µg/ml of the respective tetrapeptide (SEQ ID NO:1 - SEQ ID NO:4 and SEQ ID NO:11) or with a combination of all four tetrapeptides (SEQ ID NO:1,2,3,11). Controls received PBS. Cells were harvested after 24 hours incubation and RNA was isolated. Expression of FN1 was determined by quantitative PCR. 18s rRNA served as house keeping gene for normalization. Given are means of three independent experiments.

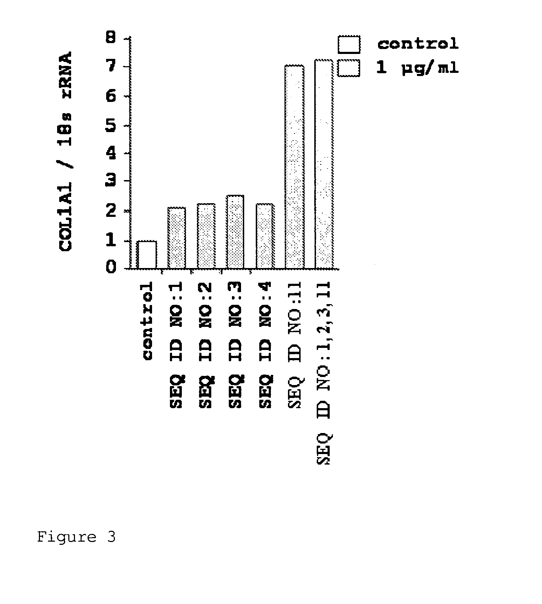

Figure 3: Collagen I alpha-1 (COL1A1) expression in human dermal fibroblasts (HDFs). HDFs were incubated with 1 µg/ml of the respective tetrapeptide (SEQ ID NO:1 - SEQ ID NO:4 and SEQ ID NO:11) or with a combination of all four tetrapeptides (SEQ ID NO:1,2,3,11). Controls received PBS. Cells were harvested after 24 hours incubation and RNA was isolated. Expression of FN1 was determined by quantitative PCR. 18s rRNA served as house keeping gene for normalization. Given are means of three independent experiments.

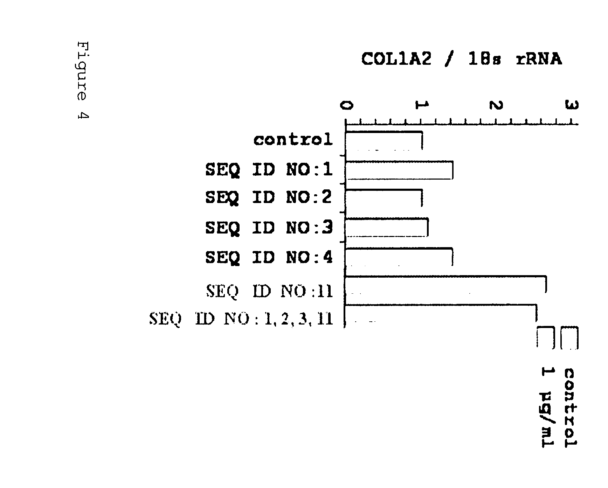

Figure 4: Collagen I alpha-2 (COL1A2) expression in human dermal fibroblasts (HDFs). HDFs were incubated with 1 µg/ml of the respective tetrapeptide (SEQ ID NO:1 - SEQ ID NO:4 and SEQ ID NO:11) or with a combination of all four tetrapeptides (SEQ ID NO:1,2,3,11). Controls received PBS. Cells were harvested after 24 hours incubation and RNA was isolated. Expression of FN1 was determined by quantitative PCR. 18s rRNA served as house keeping gene for normalization. Given are means of three independent experiments.

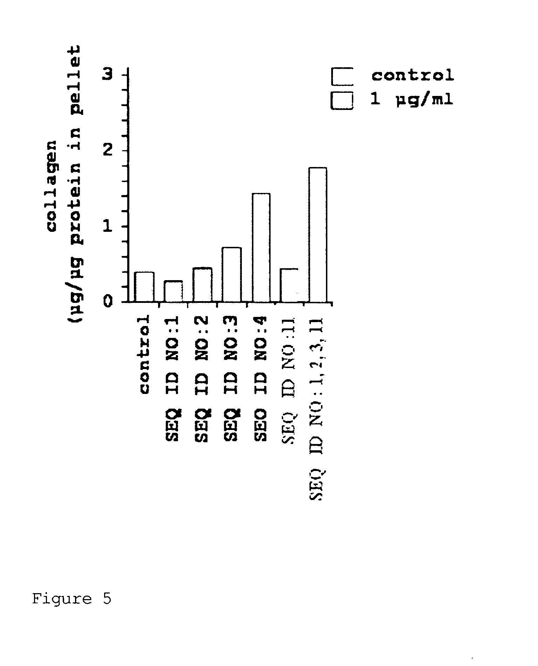

Figure 5: Collagen production in human dermal fibroblasts (HDFs). HDFs were incubated with 1 µg/ml of the respective tetrapeptide (SEQ ID NO:1 - SEQ ID NO:4 and SEQ ID NO:11) or with a combination of all four tetrapeptides (SEQ ID NO:1,2,3,11). Controls received PBS. Cell culture supernatands were collected after 24 hours incubation. Amounts of collagen were determined as described above. Given are means of three independent experiments.

### Example 1:

The effect of SEQ ID NO:1 to SEQ ID NO:4 or SEQ ID NO:11 alone or the combination of SEQ ID NO:1-3 and 11 (SEQ ID NO:1,2,3,11) containing 25% of each of tetrapeptides were investigated in-vitro using human dermal fibroblast (HDF) cell cultures. mRNA expression of fibronectin (FN1), hyaluronic acid synthetase (HAS1), collagen 1 type α1 (COL1A1) and COL1A2 and collagen production were utilized as parameters to determine extracellular matrix (ECM) molecules production.

Therefore, HDFs prepared from neonatal foreskin were cultured in EMEM (Life Technologies GmbH, Eggenstein, Germany) supplemented with 5 % fetal calf serum (Greiner, Frickenhausen, Germany), 0.1 % 1-glutamine, 2.5 % NaHCO₃, and 1 % streptomycin/amphotericin B in a humidified atmosphere containing 5 % CO₂ for 4 days until they reached confluence as described by Schieke et al. J Invest Dermatol 2002, 119: 1323-9. For all studies, only early passage (<12) fibroblasts have been used to avoid changes in their original phenotype during subculture. Cells were kept in 6well plates for culture. Peptides dissolved in PBS were added at concentrations of 1µg/ml and incubated for 24 hours.
For isolation of total RNA RNeasy Total RNA Kits (Qiagen, Hilden; Germany) were used according to the manufacturer's guidelines. The RNA concentration was determined via photometric measurement at 260/280 (Biophotometer, Eppendorf, Hamburg, Germany).
Aliquots of total RNA (100 ng) were applied for cDNA-Synthesis using Superscript^{™} III First-Strand synthesis system for the reverse transcription step with random healers (Invitrogen, Karlsruhe, Germany). For each gene, a specific primer pair was designed by Primer Express^{™} 2.0 software (Applied Bio systems, Darmstadt, Germany) based on the cDNA sequence published as indicated (Table 1). The respective gene expression was normalized using 18S rRNA as house keeping gene. Three independent experiments were performed with 3 determinations each and the mean value of these was calculated. The PCR reactions were carried out on an Option 1 (MJ Research, Waltham, MA, USA) using SYBR Green^{®} PCR Master Mix (Applied Bio systems, Darmstadt, Germany). Each sample was analyzed in double employing the universal protocol over 46 cycles. In detail, 10 minutes 94° C activation of hot start taw polymerase, 20 seconds 95° C penetration, 20 seconds 55° C annealing, 30 seconds 72° C extension. For comparison of relative expression in real time PCR control cells and treated cells the 2 (-delta delta C(T)) method was used.

The amount of collagen in cell culture supernatants was determined utilizing a Sircol^{™} Soluble Collagen Assay (# S1000) from Biocolor Ltd. (Newtownabbay, Northern Ireland, UK) following the guidelines of the manufacturer.

**Table 1: Primer sequences**

| gene | Primer sequence |
|---|---|
| FN1 | 5'-GAAAGTACACCTGTTGTCATTCAACA-3' |
| | 5'-ACCTTCACGTCTGTCACTTCCA-3' |
| HAS1 | 5'-GCGGGCTTGTCAGAGCTACT-3' |
| | 5'-AACTGCTGCAAGAGGTTATTCCTATAT-3' |
| COL1A1 | 5'-CCTGCGTGTACCCCACTCA-3' |
| | 5'-ACCAGACATGCCTCTTGTCCTT-3' |
| COL1A2 | 5'-GATTGAGACCCTTCTTACTCCTGAA-3' |
| | 5'-GGGTGGCTGAGTCTCAAGTCA-3' |
| 18S rRNA | 5'-GCCGCTAGAGGTGAAATTCTTG-3' |
| | 5'-CATTCTTGGCAAATGCTTTCG'-3' |

FN1 is a glycoprotein that is produced by many cells most prominently by fibroblasts and guarantees attachment of these cells to ECM by linking these cells to collagen fibres. Therefore, FN1 is one of the major factors involved in strength and elasticity of the skin. Here we demonstrate that treated with each of the tetrapeptides as well as with the combination of these (SEQ ID NO:1,2,3,11) induced marked up-regulation of FN1 expression with strongest effects observed for SEQ ID NO:2 and SEQ ID NO:4 (Figure 1).

Furthermore, considerable up-regulation of HAS1 expression in HDFs treated with each of the tetra peptides tested was found as well as for the combination with strongest effects for SEQ ID NO:1 and SEQ ID NO:3 as demonstrated in Figure 2. This enzyme is involved in the generation of hyaluronic acid (HA), a non-sulfated glycosaminoglycan distributed widely throughout connective, epithelial, and neural tissues. HA represents one of the main components of ECM and has been reported to contribute significantly to cell proliferation and migration.

Collagen is the main connective tissue protein that accounts for 25% of the animal protein content. Collagen has a triple-stranded rope-like coiled structure. The major collagen found in skin, tendon, and bone is the same protein containing 2 alpha-1 polypeptide chains (COL1A1) and 1 alpha-2 chain. Each of the tetrapeptides induced up-regulation of expression of COL1A1 in fibroblasts. Strongest effects were observed for SEQ ID NO:11 and the combination of the tetrapeptides SEQ ID NO:1,2,3,11 demonstrating synergistic effects for the combination (Figure 3). With respect to the expression of COL1A2 triggering effects were observed for SEQ ID NO: 1 and SEQ ID NO:4 and even more pronounced for SEQ ID NO: 11 and the combination SEQ ID NO:1,2,3,11 (Figure 4).

Since formation of the collagen triple helix, the common form of the collagen fibres, requires various steps of post-translational modifications including glycosylation and derivatisation of the amino acids, we further determined collagen protein concentration in cell culture supernatants. In accordance to the COL1A1 and COL1A2 expression data strongest effects on collagen production for the tetrapeptide combination of the tetrapeptides SEQ ID NO:1,2,3,11 was observed (Figures 5). Furthermore, treatment with SEQ ID NO:3 and SEQ ID NO:4 achieved markedly increased production of collagen by fibroblasts.

## Claims

1. Topical personal care and skin care compositions comprising:
a) a safe and effective amount of a tetrapeptide selected from the group of tetrapeptides with the motif
PX¹X²P or
PX¹X²K
where X¹ and X² are different amino acids selected from the group consisting of E, G, P and K,
the group of GEPG (SEQ ID NO: 1) peptide, GPPG (SEQ ID NO: 2) peptide, GEKG (SEQ ID NO: 3) peptide, acyl-derivatives thereof, and mixtures thereof;
b) a safe and effective amount of at least one additional active ingredient selected from the group consisting of hydroxy acids, radical scavengers, UV absorbers, vitamins, anti-aging actives, barrier lipids, desquamatory actives, retinoids, anti-acne actives, tanning actives, anti-cellulite actives, anti-inflammatory actives, antimicrobial actives, antifungal actives, skin lightening agents, skin energizing agents, skin healing agents, moisturizing actives, and mixtures thereof; and
c) a dermatologically acceptable carrier.

2. The topical composition of claim 1, wherein the tetrapeptide is used in amounts from about 1 ppm to about 1000 ppm, preferably from about 2 ppm to about 250 ppm, and most preferably from about 10 ppm to about 100 ppm by weight of the composition.

3. The topical composition of any of claims 1 or 2, wherein the tetrapeptide is selected from the group of GEPG (SEQ ID NO: 1) peptide, GPPG (SEQ ID NO: 2) peptide, GEKG (SEQ ID NO: 3) peptide, PGPP (SEQ ID NO: 4) peptide, and PKEK (SEQ ID NO: 5) peptide.

4. The topical composition of any of claims 1 to 3, wherein N-acyl-GEPG (SEQ ID NO: 6) peptide, N-acyl-GPPG (SEQ ID NO: 7) peptide, N-acyl-GEKG (SEQ ID NO: 8) peptide, N-acyl-PGPP (SEQ ID NO: 9) peptide and/or N-acyl-PKEK (SEQ ID NO: 10) peptide are used as tetrapeptide.

5. The topical composition of any of the preceding claims wherein the topical composition further comprises a GAGP (SEQ ID NO: 11) tetrapeptide.

6. The topical composition of any of the preceding claims wherein the topical composition comprises from about 10 ppm to about 100 ppm, by weight of the composition , of either GEPG, GPPG, GEKG, PKEK, or PGPP; a dermatologically acceptable carrier and at least one additional active ingredient selected from
a) from about 0.05% to about 1.5%, by weight of the composition, of creatine;
b) from about 0.01% to about 1.0%, by weight of the composition, of ceramides and/or sphingolipids;
c) from about 0.01% to about 0.5%, by weight of the composition, of a hyaluronic acid and/or beta glucane;
d) from about 0.05% to about 1.0%, by weight of the composition, of arjunolic acid and/or turmeric oil.

7. A method of reducing the visible signs of aging in skin and/or hair comprising: applying to skin or hair in need of such treatment the topical composition of any of claims 1 to 6 at least once a day over an extended period of time.

## Patentansprüche

1. Topische Personal-Care- und Hautpflegezusammensetzungen, die Folgendes umfassen:
a) eine ungefährliche und wirksame Menge eines Tetrapeptids, ausgewählt
aus der Gruppe der Tetrapeptide mit dem Motiv
PX¹X²P oder
PX¹X²K,
worin X¹ und X² unterschiedliche Aminosäuren, ausgewählt aus
der Gruppe bestehend aus E, G, P und K bedeuten, der Gruppe GEPG (SEQ ID NO: 1)-Peptid, GPPG (SEQ ID NO: 2)-Peptid, GEKG (SEQ ID NO: 3)-Peptid, Acylderivaten davon und Mischungen davon;
b) eine ungefährliche und wirksame Menge an mindestens einem zusätzlichen Wirkstoff, ausgewählt aus der Gruppe bestehend aus Hydroxysäuren, Radikalfängern, UV-Absorbern, Vitaminen, Anti-Ageing-Wirkstoffen, Barrierelipiden, Desquamationswirkstoffen, Retinoiden, Wirkstoffen gegen Akne, Bräunungswirkstoffen, Wirkstoffen gegen Cellulite, entzündungshemmenden Wirkstoffen, antimikrobiellen Wirkstoffen, antifungalen Wirkstoffen, Hautaufhellungsmitteln, Wirkstoffen, die der Haut Energie spenden, hautheilenden Wirkstoffen, feuchtigkeitsspendenden Wirkstoffen und Mischungen davon; sowie
c) einen dermatologisch unbedenklichen Träger.

2. Topische Zusammensetzung nach Anspruch 1, wobei das Tetrapeptid in Mengen von ungefähr 1 ppm bis ungefähr 1000 ppm, vorzugweise von ungefähr 2 ppm bis ungefähr 250 ppm und am stärksten bevorzugt von ungefähr 10 ppm bis ungefähr 100 ppm in Bezug auf das Gewicht der Zusammensetzung eingesetzt wird.

3. Topische Zusammensetzung nach einem der Ansprüche 1 oder 2, wobei das Tetrapeptid aus der Gruppe GEPG (SEQ ID NO: 1)-Peptid, GPPG (SEQ ID NO: 2)-Peptid, GEKG (SEQ ID NO: 3) -Peptid, PGPP (SEQ ID NO: 4)-Peptid und PKEK (SEQ ID NO: 5)-Peptid ausgewählt ist.

4. Topische Zusammensetzung nach einem der Ansprüche 1 bis 3, wobei N-Acyl-GEPG (SEQ ID NO: 6) -Peptid, N-Acyl-GPPG (SEQ ID NO: 7)-Peptid, N-Acyl-GEKG (SEQ ID NO: 8)-Peptid, N-Acyl-PGPP (SEQ ID NO: 9)-Peptid und/oder N-Acyl-PKEK (SEQ ID NO: 10)-Peptid als Tetrapeptid eingesetzt werden.

5. Topische Zusammensetzung nach einem der vorhergehenden Ansprüche, wobei die topische Zusammensetzung weiterhin ein GAGP (SEQ ID NO: 11)-Tetrapeptid umfasst.

6. Topische Zusammensetzung nach einem der vorhergehenden Ansprüche, wobei die topische Zusammensetzung von ungefähr 10 ppm bis ungefähr 100 ppm in Bezug auf das Gewicht der Zusammensetzung an einem von GEPG, GPPG, GEKG, PKEK oder PGPP; einen dermatologisch unbedenklichen Träger und mindestens einen zusätzlichen Wirkstoff, ausgewählt aus Folgendem:
a) Kreatin in einer Menge von ungefähr 0,05% bis ungefähr 1,5% in Bezug auf das Gewicht der Zusammensetzung;
b) Ceramide und/oder Sphingolipide in einer Menge von ungefähr 0,01% bis ungefähr 1,0% in Bezug auf das Gewicht der Zusammensetzung;
c) eine Hyaluronsäure und/oder ein beta-Glucan in einer Menge von ungefähr 0,01% bis ungefähr 0,5% in Bezug auf das Gewicht der Zusammensetzung;
d) Arjunolsäure und/oder Kurkumaöl in einer Menge von ungefähr 0,05% bis ungefähr 1,0% in Bezug auf das Gewicht der Zusammensetzung,
umfasst.

7. Verfahren zum Verringern der sichtbaren Anzeichen von Haut- und/oder Haaralterung, bei dem man über einen längeren Zeitraum mindestens einmal täglich die topische Zusammensetzung nach einem der Ansprüche 1 bis 6 auf die Haut oder das Haar, die/das solch einer Behandlung bedarf, aufträgt.

## Revendications

1. Compositions topiques pour les soins personnels et les soins de la peau, comprenant :
a) une quantité sûre et efficace d'un tétrapeptide choisi dans le groupe de tétrapeptides présentant le motif PX¹X²P ou PX¹X²K, où X¹ et X² sont des acides aminés différents, choisis dans le groupe consistant en E, G, P et K, le groupe du peptide GEPG (SEQ ID N° 1), du peptide GPPG (SEQ ID N° 2), du peptide GEKG (SEQ ID N° 3), les dérivés acylés de ceux-ci et les mélanges de ceux-ci ;
b) une quantité sûre et efficace d'au moins un principe actif additionnel choisi dans le groupe consistant en les hydroxyacides, les piégeurs de radicaux, les absorbants UV, les vitamines, les actifs anti-vieillissement, les lipides barrières, les actifs desquamants, les rétinoïdes, les actifs anti-acnéiques, les actifs bronzants, les actifs anti-cellulite, les actifs anti-inflammatoires, les actifs antimicrobiens, les actifs antifongiques, les agents d'éclaircissement de la peau, les actifs d'excitation de la peau, les agents de cicatrisation de la peau, les actifs humectants et les mélanges de ceux-ci ; et
c) un support dermatologiquement acceptable.

2. Composition topique de la revendication 1, dans laquelle le tétrapeptide est utilisé en des quantités d'environ 1 ppm à environ 1 000 ppm, de préférence d'environ 2 ppm à environ 250 ppm et tout spécialement d'environ 10 ppm à environ 100 ppm en poids par rapport à la composition.

3. Composition topique de l'une quelconque des revendications 1 ou 2, dans laquelle le tétrapeptide est choisi dans le groupe du peptide GEPG (SEQ ID N° 1), du peptide GPPG (SEQ ID N° 2), du peptide GEKG (SEQ ID N° 3), du peptide PGPP (SEQ ID N° 4) et du peptide PKEK (SEQ ID N° 5).

4. Composition topique de l'une quelconque des revendications 1 à 3, dans laquelle on utilise en tant que tétrapeptide le peptide N-acyl-GEPG (SEQ ID N° 6), le peptide N-acyl-GPPG (SEQ ID N° 7), le peptide N-acyl-GEKG (SEQ ID N° 8), le peptide N-acyl-PGPP (SEQ ID N° 9) et/ou le peptide N-acyl-PKEK (SEQ ID N° 10).

5. Composition topique de l'une quelconque des revendications précédentes, la composition topique comprenant en outre un tétrapeptide GAGP (SEQ ID N° 11).

6. Composition topique de l'une quelconque des revendications précédentes, la composition topique comprenant d'environ 10 ppm à environ 100 ppm en poids, par rapport à la composition, du GEPG, du GPPG, du GEKG, du PKEK ou du PGPP ; un support dermatologiquement acceptable et au moins un principe actif additionnel choisi parmi
a) d'environ 0,05 % à environ 1,5 % en poids de créatine par rapport à la composition ;
b) d'environ 0,01 % à environ 1,0 % en poids de céramides et/ou de sphingolipides par rapport à la composition ;
c) d'environ 0,01 % à environ 0,5 % en poids d'acide hyaluronique et/ou de bêta-glucane par rapport à la composition ;
d) d'environ 0,05 % à environ 1,0 % en poids d'acide arjunolique et/ou d'huile de curcuma par rapport à la composition.

7. Procédé de réduction des signes visibles de vieillissement de la peau et/ou des cheveux, comprenant l'application, sur la peau ou les cheveux qui ont besoin d'un tel traitement, au moins une fois par jour ou sur un période de temps étendue, de la composition topique de l'une quelconque des revendications 1 à 6.


### SEQUENCE LISTING

<110> Evonik Goldschmidt GmbH
<120> Personal care and cosmetic composition containing tetrapeptides with the motifs GX1X2G, PX1X2P, or PX1X2K
<130> 2007P00830
<160> 11
<170> PatentIn version 3.4
<210> 1
   <211> 4
   <212> PRT
   <213> artificial
<220>
   <223> Description of Artificial Sequence: Gly Glu Pro Gly
<400> 1
   Gly Glu Pro Gly
   1
<210> 2
   <211> 4
   <212> PRT
   <213> artificial
<220>
   <223> Description of Artificial Sequence: Gly Pro Pro Gly
<400> 2
   Gly Pro Pro Gly
   1
<210> 3
   <211> 4
   <212> PRT
   <213> artificial
<220>
   <223> Description of Artificial Sequence: Gly Glu Lys Gly
<400> 3
   Gly Glu Lys Gly
   1
<210> 4
   <211> 4
   <212> PRT
   <213> artificial
<220>
   <223> Description of Artificial Sequence: Pro Gly Pro Pro
<400> 4
   Pro Gly Pro Pro
   1
<210> 5
   <211> 4
   <212> PRT ,
   <213> artificial
<220>
   <223> Description of Artificial Sequence: Pro Lys Glu Lys
<400> 5
   Pro Lys Glu Lys
   1
<210> 6
   <211> 4
   <212> PRT
   <213> artificial
<220>
   <223> Description of Artificial Sequence: N-acyl-Gly Glu Pro Gly
<400> 6
   Gly Glu Pro Gly
   1
<210> 7
   <211> 4
   <212> PRT
   <213> artificial
<220>
   <223> Description of Artificial Sequence: N-acyl-Gly Pro Pro Gly
<400> 7
   Gly Pro Pro Gly
   1
<210> 8
   <211> 4
   <212> PRT
   <213> artificial
<220>
   <223> Description of Artificial Sequence: N-acyl-Gly Glu Lys Gly
<400> 8
   Gly Glu Lys Gly
   1
<210> 9
   <211> 4
   <212> PRT
   <213> artificial
<220>
   <223> Description of Artificial Sequence: N-acyl-Pro Gly Pro Pro
<400> 9
   Pro Gly Pro Pro
   1
<210> 10
   <211> 4
   <212> PRT
   <213> artificial
<220>
   <223> Description of Artificial Sequence: N-acyl-Pro Lys Glu Lys
<400> 10
   Pro Lys Glu Lys
   1
<210> 11
   <211> 4
   <212> PRT
   <213> artificial
<220>
   <223> Description of Artificial Sequence: Gly Ala Gly Pro
<400> 11
   Gly Ala Gly Pro
   1